(19) Europäisches Patentamt
European Patent Office
Office européen des brevets

(11) Veröffentlichungsnummer: **0 006 152**
**B1**

(12) # EUROPÄISCHE PATENTSCHRIFT

(21) Anmeldenummer: **79101627.2**

(22) Anmeldetag: **28.05.79**

(51) Int. Cl.³: **C 07 C 131/00**

(54) **Verfahren zur Herstellung von 2-Oximinophenylacetonitril**

(30) Priorität: **10.06.78 DE 2825565**

(43) Veröffentlichungstag der Anmeldung:
**09.01.80 Patentblatt 80/01**

(45) Bekanntmachung des Hinweises auf die Patenterteilung:
**12.11.80 Patentblatt 80/23**

(84) Benannte Vertragsstaaten:
**BE CH DE FR GB IT NL**

(56) Entgegenhaltungen:
**HOUBEN-WEYL "Methoden der organischen Chemie",
4, Auflage, Band X, Teil 4, 1968,
GEORG THIEME VERLAG, Stuttgart Seiten 31, 32**

(73) Patentinhaber: **BAYER AG
ZENTRALBEREICH PATENTE,
MARKEN UND LIZENZEN
D-5090 LEVERKUSEN 1,
BAYERWERK (DE)**

(72) Erfinder: **Schröder, Horst, Dr.
9022 Birch
Prairie Village Kansas 66207 (US)
Goliasch, Karl, Dr.
Drosselweg 37
D-5060 Berg.Gladbach 2 (DE)
Beck, Uwe, Dr.
Wolfskaul 10
D-5000 Köln 80 (DE)**

**EP 0 006 152 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von 2-Oximinophenylacetonitril

Die vorliegende Erfindung betrifft ein neues Verfahren zur Herstellung von 2-Oximinophenylacetonitril, das als Zwischenprodukt für die Synthese von pestiziden Wirkstoffen verwendet werden kann.

Die Herstellung von 2-Oximinophenylacetonitril ist bekannt. Eine umfassende Übersicht über die Herstellungsverfahren ist in Houben-Weyl, Methoden der organischen Chemie, Bd. X/4 (1968), 4. Auflage erschienen. Danach läßt sich 2-Oximinophenylacetonitril beispielsweise auch durch Umsetzung von Benzylcyanid, Alkylnitrit und Natriumalkoholat in wasserfreiem Alkohol herstellen (s. auch T. E. Stevenson, J.org.Chem. *28,* 2436 (1963) und J. T. Thurston, J.org.Chem. *2,* 192 (1938).

Es wurde nun gefunden, daß man 2-Oximinophenylacetonitril durch Umsetzung von Benzylcyanid mit Alkylnitrit in Gegenwart einer Base erhält, wenn man in eine Mischung aus einem Alkohol, einer wäßrigen Lösung einer anorganischen Base und Benzylcyanid ein Alkylnitrit eindosiert, nach beendeter Reaktion die Lösung des 2-Oximinophenylacetonitril-Basensalzes ansäuert und danach das 2-Oximinophenylacetonitril isoliert.

Der Reaktionsablauf des erfindungsgemäßen Verfahrens kann durch folgendes Formelschema wiedergegeben werden:

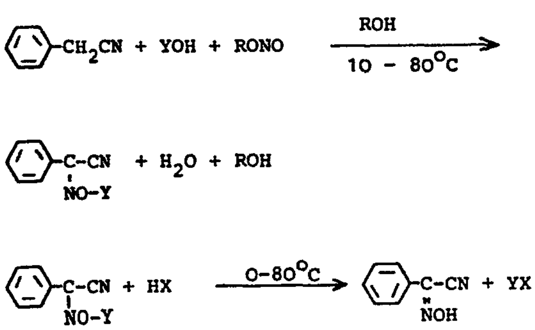


Dabei bedeuten Y—OH eine starke anorganische Base, HX eine Mineralsäure und R einen Alkylrest.

Es ist als ausgesprochen überraschend zu bezeichnen, daß gemäß der erfindungsgemäßen Umsetzung anstelle von teurem, schwer zu handhabendem Alkalialkoholat mit sehr gutem Erfolg auch konzentrierte, wäßrige, Lösungen von anorganischen Basen und wasserhaltiger Alkohol als Lösungsmittel eingesetzt werden können.

Nach dem erfindungsgemäßen Verfahren können verschiedene Alkylnitrite Verwendung finden. Bevorzugt werden die niederen Homologen $CH_3ONO$ bis $C_5H_{11}ONO$ wegen ihrer höheren Reaktionsgeschwindigkeit eingesetzt.

Das Molverhältnis von Benzylcyanid zum Alkylnitrit kann 1:1 bis 1:1,2, bevorzugt 1:1,05 bis 1:1,15, betragen.

Der Alkylrest des als Lösungsmittel eingesetzten Alkohols ist ohne Bedeutung für die Reaktion. Der Alkylrest sollte aber mit dem des Alkylnitrites übereinstimmen, um eine spätere Aufarbeitung von Lösungen zu erleichtern.

Als Basen kommen alle starken, anorganischen Basen in Frage. Vorzugsweise werden wäßrige Lösungen von Natriumhydroxid oder Kaliumhydroxid eingesetzt.

Wird die Umsetzung mit Alkalilauge ausgeführt, kann das Molverhältnis von Benzylcyanid zum Alkalihydroxid 1:1 bis 1:1,5, bevorzugt 1:1,1 bis 1:1,3, betragen.

Die Oximierungs-Reaktion läuft exotherm ab. Die Reaktion nach dem erfindungsgemäßen Verfahren kann bei 10 bis 80°C, vorzugsweise zwischen 20 bis 40°C, durchgeführt werden.

Die Hauptreaktion ist innerhalb weniger Minuten abgeschlossen. Sie ist abhängig von der Art des eingesetzten Alkylnitrites und von der Reaktionsführung.

Zur Neutralisation des alkalischen Reaktionsgemisches kann jede starke Säure Verwendung finden. Vorzugsweise können technische Salzsäure unde Schwefelsäure eingesetzt werden Die Neutralisation kann bei Temperaturen von 0 bis 80°C, vorzugsweise zwischen 40 bis 60°C, durchgeführt werden.

Nach Abkühlen, nach Verdünnen mit kaltem Wasser oder nach Abdestillation des Alkohols kristallisiert das Oximinophenylacetonitril aus und kann durch Filtration isoliert werden.

Das beschriebene Verfahren ermöglicht die Herstellung von 2-Oximinophenylacetonitril ohne Isolierung von Zwischenprodukten.

Die Reaktionsführung kann mit gutem Erfolg kontinuierlich und diskontinuierlich gestaltet werden. Besonders bevorzugt ist eine kontinuierliche Reaktionsführung, dergemäß die beispielsweise aus Methonal, Natronlauge und Benzylcyanid bestehende Reaktionslösung im Kreis gepumpt wird und dabei einen Injektor betreibt, der beispielsweise Methylnitrit ansaugt.

Nach dem erfindungsgemäßen Verfahren erhält man 2-Oximinophenylacetonitril in großer Reinheit und guter Ausbeute. 2-Oximinophenylacetonitril eignet sich zur Herstellung wertvoller Phosphor-, Phosphon- bzw. Thionophosphor-, -phosophonsäureester, die mit Erfolg im Pflanzenschutz zur Bekampfung schädlicher saugender und beißender Insekten, Dipteren und Milben (Acarina) sowie auf dem Veterinär- und Hygienesektor, ferner im Vorratsschutz gegen eine Vielzahl von tierischen Schädlingen (Endo- und Ektoparasiten) eingesetzt werden können (s. z.B. DE-PS 1 238 902).

## Beispiel 1

In eine Lösung aus 980 g Methanol, 480 g Natronlauge, 50 %ig und 468 g Benzylcyanid werden bei 35 bis 45°C innerhalb 2 bis 4 Stunden 290 g Methylnitrit eingeleitet. Eine halbe

Stunde bei 35 bis 45°C nachrühren und mit 7 l Eiswasser verdünnen. Der Ansatz wird mit Salzsäure auf pH 2 bis 3 gestellt. Das gebildete Oximinophenylacetonitril wird abfiltriert und mit Wasser gewaschen.

Ausbeute: 545 g Oximinophenylacetonitril (bestimmt aus dem Rohprodukt).

Beispiel 2

In eine Mischung aus 70 g Isopropanol, 130 g Natronlauge 40 %ig und 114 g Benzylcyanid werden bei 25 bis 35°C innerhalb von 8 bis 10 Stunden 105 g Isopropylnitrit gegeben.

Der Ansatz wird mit Salzsäure auf pH 2 bis 3 gestellt.

Überschüssiges Isopropylnitrit und Isopropanol werden im Vakuum abdestilliert.

Das gebildete Oximinophenylacetronitril wird bei 20°C abfiltriert und mit Wasser gewaschen.

Ausbeute: 122 g Oximinophenylacetonitril (bestimmt aus dem Rohprodukt).

Beispiel 3

In eine Lösung aus 475 g Methanol, 215 g Wasser, 126 g Natriumhydroxid und 279 g Benzylcyanid werden bei 30 bis 40°C innerhalb von 4 bis 5 Stunden 167 g Methylnitrit eingeleitet. Eine halbe Stunde bei 35°C nachrühren. Salzsäure bis pH 2 bis 3 zufügen und die Temperatur auf 55 bis 60°C steigen lassen.

Der Ansatz wird innerhalb 1/2 Stunde auf 4 l Eiswasser ausgetragen. Das gebildete Oximinophenylacetonitril wird abfiltriert und mit Wasser gewaschen.

Ausbeute: 317 g Oximinophenylacetonitril (bestimmt aus dem Rohprodukt).

Beispiel 4

200 kg/h Methanol, 80 kg/h Natronlauge 50 %, 60 kg/h Wasser und 94 kg/h Benzylcyanid werden bei 30 bis 40°C miteinander umgesetzt. Die Reaktionslösung wird im Kreis gepumpt und betreibt dabei einen Injektor, der 54 kg/h Methylnitrit ansaugt.

Entsprechend dem Zulauf der Einsatzprodukte wird ein Teil des Reaktionsgemisches abgenommen.

Die Reaktion wird drucklos durchgeführt.

Nach Verdünnen mit Wasser und Ansäuern mit Schwefelsäure wird das gebildete Oximinophenylacetonitril abfiltriert und mit Wasser gewaschen.

Ausbeute: 103 bis 108 kg/h Oximinophenylacetonitril (bestimmt aus dem Rohprodukt).

**Patentansprüche**

1. Verfahren zur Herstellung von 2-Oximinophenylacetonitril durch Umsetzung von Benzylcyanid mit Alkylnitrit in Gegenwart einer Base, dadurch gekennzeichnet, daß man in eine Mischung aus einem Alkohol, einer wäßrigen Lösung einer anorganischen Base und Benzylcyanid ein Alkylnitrit eindosiert, nach beendeter Reaktion die Lösung des 2-Oximinophenylacetonitril-Basensalzes ansäuert und danach das 2-Oximinophenylacetonitril isoliert.

2. Verfahren nach Anspruch 1, dadurch gekennzeichnet, daß auf 1 Mol Benzylcyanid 1 bis 1,2 Mol Alkylnitrit in das Reaktionsgemisch eindosiert werden.

3. Verfahren nach Anspruch 1 und 2, dadurch gekennzeichnet, daß als starke anorganische Basen wäßrige Lösungen von Kalium- oder Natriumhydroxid im Molverhältnis zum Benzylcyanid von 1:1 bis 1,5:1 verwendet werden.

4. Verfahren nach den Ansprüchen 1 bis 3, dadurch gekennzeichnet, daß $C_1$—$C_5$-Alkylnitrite verwendet werden.

5. Verfahren nach den Ansprüchen 1 bis 4, dadurch gekennzeichnet, daß das alkalische Reaktionsgemisch nach beendeter Umsetzung mit Salz- oder Schwefelsäure bei Temperaturen zwischen 40° bis 60°C angesäuert wird und das gebildete 2-Oximinophenylacetonitril abgetrennt wird.

6. Verfahren nach Anspruch 1, dadurch gekennzeichnet, daß das aus einem Alkohol, einer wäßrigen Lösung einer anorganischen Base und Benzylcyanid bestehende Reaktionsgemisch im Kreis gepumpt wird und dabei einen Injektor betreibt, der das Alkylnitrit ansaugt.

7. Verfahren nach Anspruch 1 bis 6, dadurch gekennzeichnet, daß die Reaktionstemperatur 10 bis 80°C beträgt.

**Revendications**

1. Procédé de production de 2-oximinophénylacétonitrile par réaction de cyanure de benzyle avec un nitrite d'alkyle en présence d'une base, caractérisé en ce qu'on introduit un nitrite d'alkyle dans un mélange d'un alcool, d'une solution aqueuse d'une base inorganique et de cyanure de benzyle, on acidifie la solution de sel de base de 2-oximinophénylacétonitrile lorsque la réaction est terminée puis on isole le 2-oximinophénylacétonitrile.

2. Procédé suivant la revendication 1, caracterisé en ce qu'on introduit par mole de cyanure de benzyle 1 à 1,2 mole de nitrite d'alkyle dans le mélange réactionnel.

3. Procédé suivant les revendications 1 et 2, caractérisé en ce qu'on utilise comme bases inorganiques fortes des solutions aqueuses d'hydroxyde de potassium ou d'hydroxyde de sodium dans un rapport molaire avec le cyanure de benzyle de 1:1 à 1,5:1.

4. Procédé suivant les revendication 1 à 3, caractérisé en ce qu'on utilise des nitrites d'alkyle en $C_1$ à $C_5$.

5. Procédé suivant les revendications 1 à 4, caractérisé en ce que le mélange réactionnel alcalin est acidifié, lorsque la réaction est terminée, avec de l'acide chlorhydrique ou sulfurique à des températures allant de 40 à 60°C et on sépare le 2-oximinophénylacétonitrile formé.

6. Procédé suivant la revendication 1,

caracterisé en ce que le mélange réactionnel formé d'un alcohl, d'une solution aqueuse d'une base inorganique et de cyanure de benzyle circule sous l'action d'une pompe et actionne ainsi un injecteur qui aspire le nitrite d'alkyle.

7. Procédé suivant les revendications 1 à 6, caractérisé en ce que la température de réaction a une valeur de 10 à 80°C.

**Claims**

1. Process for the preparation of 2-oximiniophenylacetonitrile by reacting benzyl cyanide with an alkyl nitrite in the presence of a base, characterised in that an alkyl nitrite is metered into a mixture of an alcohol, an aqueous solution of an inorganic base and benzyl cyanide, the solution of the 2-oximinophenylacetonitrile base salt is acidified after the reaction has ended and the 2-oximinophenylacetonitrile is then isolated.

2. Process according to Claim 1, characterised in that 1 to 1.2 mols of alkyl nitrite are metered into the reaction mixture per 1 mol of benzyl cyanide.

3. Process according to Claim 1 and 2, characterised in that aqueous solutions of potassium hydroxide or sodium hydroxide, in a molar ratio to the benzyl cyanide of 1:1 to 1.5:1, are used as the strong inorganic bases.

4. Process according to Claims 1 to 3, characterised in that $C_1$—$C_5$-alkyl nitrites are used.

5. Process according to Claims 1 to 4, characterised in that, after the reaction has ended, the alkaline reaction mixture is acidified with hydrochloric acid or sulphuric acid at temperatures between 40°C and 60°C and the 2-oximinophenylacetonitrile formed is separated off.

6. Process according to Claim 1, characterised in that the reaction mixture, consisting of an alcohol, an aqueous solution of an inorganic base and benzyl cyanide, is circulated by means of a pump and thereby operates an injector which sucks in the alkyl nitrite.

7. Process according to Claim 1 to 6, characterised in that the reaction temperature is 10 to 80°C.